Europäisches Patentamt

European Patent Office

Office européen des brevets

(19)

(11) Publication number: **0 038 080**
**B1**

(12) **EUROPEAN PATENT SPECIFICATION**

(45) Date of publication of patent specification: **28.08.85**

(21) Application number: **81102888.5**

(22) Date of filing: **15.04.81**

(51) Int. Cl.⁴: **A 61 N 1/38**

(54) **Patient interactive stimulator.**

(30) Priority: **16.04.80 US 140745**

(43) Date of publication of application:
**21.10.81 Bulletin 81/42**

(45) Publication of the grant of the patent:
**28.08.85 Bulletin 85/35**

(84) Designated Contracting States:
**DE FR NL**

(56) References cited:
**DE-A-2 822 799**
**DE-A-3 035 732**
**FR-A-2 305 197**
**FR-A-2 369 836**
**GB-A-1 467 344**
**US-A-3 805 795**
**US-A-3 871 361**
**US-A-3 942 536**
**US-A-4 102 332**
**US-A-4 146 029**
**US-A-4 210 149**

(73) Proprietor: **MEDTRONIC, INC.**
**3055 Old Highway Eight P.O. Box 1453**
**Minneapolis Minnesota 55440 (US)**

(72) Inventor: **Engle, William R.**
**605 Harriet Avenue Appartment No. 413**
**St. Paul, MN 55112 (US)**

(74) Representative: **Schwan, Gerhard, Dipl.-Ing.**
**Elfenstrasse 32**
**D-8000 München 83 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates generally to a body-implantable therapy device, for example to a body-implantable automatic cardioverting device.

Recently, body implantable devices have been developed to automatically treat life-threatening conditions, particularly arrhythmic heart conditions. For example, an automatic cardioverting device is disclosed in US—A—3 805 795. This device monitors at least one heart function. If there is no sensing of a dynamic characteristic indicative of a normal functioning heart during a specified period of time, for example, 20 seconds, it is assumed the patient's heart has developed a life-threatening arrhythmic condition and an electrical heart stimulating pulse is automatically delivered to the patient's heart. It should be noted that this device includes an alert system comprising both a visual and an audio alarm which is activated prior to the actual therapy. Furthermore a body-implantable therapy device, particularly a permanently implanted cardioverter, comprising body function monitoring means for generating a signal indicating the apparent existence of a condition requiring therapy, means for administering therapy appropriate for the condition, control means operable by the patient for controlling said therapy means to administer or not to administer therapy, and means for alerting a patient to imminent operation of said therapy means in response to a signal from the monitoring means indicating the apparent existence of a condition requiring therapy is known from DE—A—2 822 799. This device enables the patient to discharge the defibrillation energy pulse through an implanted test load to verify the operation of the cardioversion portion of the circuitry.

It will be apparent that treatment with the aid of a device of the aforementioned type is a relatively traumatic procedure, and a false indication leading to inappropriate treatment is highly undesirable.

With respect to an automatic implantable defibrillator, there are a number of possible occurrences which might, if not otherwise prevented, inappropriately cause a defibrillating pulse to be delivered to the heart. Two examples are exposure to an external electromagnetic field such as is emitted by certain types of security devices; and even a malfunction in the heart monitoring portion of the implantable cardioverting device itself. Whatever the reason, inappropriate treatment, particularly by a defibrillator, is highly undesirable. Not only would such inappropriate treatment be quite painful, but it might actually stop an otherwise normally functioning heart.

In contrast to the device of the present invention which is intended to automatically treat severe life-threatening conditions, such as an arrhythmic heart condition known as ventricular fibrillation, by relatively traumatic and potentially dangerous (therefore to be administered only if actually needed) therapy, such as a strong electric shock, patient-controllable devices have been proposed for treating less severe conditions. For example, US—A—3 952 750 discloses a body-implantable therapy device which may be triggered by a patient or physician to effect cardioversion which treats less threatening arrhythmias such as atrial fibrillation, atrial flutter, or tachycardia. As described in this patent, a patient can be taught to recognize the symptoms of such arrhythmias and to effect cardioversion by holding a magnet at an appropriate location against his skin to close a reed switch. It is stated that this cardioversion can be a painless procedure not requiring anesthesia.

While the present invention is described in the context of an automatic cardiac defibrillator, the broader concepts thereof are applicable to other body implantable therapy devices such as implantable insulin dispensers or implantable cardiac demand pacemakers having a rate limit. One specific example of a device for automatic treatment by drug injection when a specific body condition occurs is disclosed in US—A—3 871 361. In this device, a timer is employed to provide some assurance that the condition which occurs in the patient requiring medication is of a sufficient duration to indicate an emergency medical situation, rather than merely a temporary change in the supervised bodily function which is of a non-emergency status.

Accordingly, it is an object of the invention to provide a body-implantable therapy device, which reduces the incidence of inappropriate treatment.

Briefly stated, and in accordance with a general concept of the invention, the foregoing and other objects are accomplished by a body-implantable therapy device of the type defined in the first part of claim 1 in that the alerting means are functioning whenever a signal from the monitoring means indicates the existence of a condition requiring therapy and in that memory means are provided giving the patient for a predetermined time the possibility to interrupt the action of said control means.

When the condition requiring therapy apparently occurs or exists, the body function monitoring means generates a signal. As previously mentioned, this signal either may properly indicate the actual existence of a condition requiring therapy, or improperly may be the result of some form of interference, or a malfunction. The patient is alerted in response to a signal from the monitoring means, and the memory means allow the patient to determine what course of action the device should take.

In one embodiment of the invention the control means after said predetermined time controls the therapy means not to administer therapy if the patient does not take a defined action in order to enable the control means to permit the therapy means to be actuated (first mode). Accordingly, the patient operable control means effects or initiates the therapy means to administer therapy to the patient, and the memory means stores the therapy condition signal for the predetermined time during which the patient may operate the control means to initiate the administering of therapy to the patient.

In another embodiment of the invention the memory means comprises delay means for triggering the control means and thereby the therapy means the predetermined time after the monitoring means has generated a signal, and the control means comprises interrupt means operable by the patient within the predetermined time for inhibiting operation of the therapy means (second mode).

Thus, in the absence of intervention by the patient, the therapy proceeds after the predetermined delay intended to give him an opportunity to interrupt the therapy. If, on the other hand, the patient acts, operation of the therapy means is inhibited.

Such a device has particular utility when employed with an automatic cardioverting device intended to treat relatively serious or lethal arrhythmic conditions, such as ventricular fibrillation. For such an instance, if the condition apparently sensed by the body function monitoring means does in fact exist, then is most cases the patient is unconscious. Thus the patient need take no action whatsoever for therapy to be automatically administered, and that is exactly what the patient is capable of doing.

In accordance with a more particular embodiment of the invention, a body-implantable automatic cardioverting device includes a heart function monitoring means for generating a signal indicating the apparent existence of a life-threatening arrhythmic condition. Further, the device includes cardioversion means for administering a cardioverting shock to the heart when triggered. There is a time delay means for triggering the cardioversion means a predetermined time after the monitoring means generates a signal. A patient alert means is responsive, along with the time delay means, to signals from the monitoring means for alerting a patient to imminent operation of the cardioversion means. Lastly, an interrupt means is operable by the patient for inhibiting operation of the cardioversion means.

From the foregoing, it will be apparent that the present invention requires two-way communication between the patient and the implanted device. Any suitable means may be employed for this purpose. For example, the means for alerting the patient to imminent operation of the therapy means may comprise a tissue stimulating device such as is disclosed in US—A—4 140 131. Specifically, this device is an implantable pacemaker which alerts the patient to various conditions such as malfunction or initial operation of a demand pacemaker by applying electrical stimulation to tissues comprising and surrounding the body pocket in which the pacemaker is placed. Suitable electrodes are provided on the case of the pacemaker for this purpose.

For communication from a patient or other person to a body-implantable device, a number of suitable prior art approaches are known. For example, the above-mentioned implantable pacemaker includes a magnetically actuated reed switch to receive communications in the form of a magnetic field from a point external to the body and to the implantable device. All that need to done to communicate a signal recognizable to the implantable device is to hold a magnet over an area of the body near the implantable device.

Another method for communicating a control signal to a body-implantable device is disclosed in the US—A—4 066 086 and comprises a receiver responsive to electromagnetic energy. In the specific example given in said patent, the device is responsive to radio frequency energy on a carrier frequency of 176 kHz.

In accordance with another embodiment of the invention, still another means for sending a communication to the body-implantable device may be employed. Specifically, the patient operable interrupt means of the body implantable automatic therapy device may include a mechanical shock sensor, such as an accelerometer type device. The patient may operate the interrupt means for the purpose of inhibiting the therapy means by striking an area of the body appropriate for transmitting a shock to the mechanical shock sensor. This approach has the advantage that no external hardware device, such as a magnet or a radio transmitter, is required, and therefore there is no possibility of an essential piece of equipment becoming lost or being unavailable when needed. All that is required is a simple blow or forceful thump to the chest or area near where the cardioverting device is implanted.

In accordance with still another embodiment of the invention, the patient interrupt means may be arranged such that periodic positive actions are required by the patient for continued inhibiting of the operation of the therapy means. This is to discourage the patient from attempting to permanently disable the device by, for example, taping a magnet in place over the implanted device for the purpose of avoiding a supposed nuisance.

While the features of the invention are set forth with particularity in the appended claims, the invention, both as to organization and content, will be better understood and appreciated, along with other objects and features of its embodiments, from the following detailed description taken in conjunction with the drawings, in which:

FIG. 1 is a block diagram illustrative of the general concepts of the present invention;

FIG. 2 is a partially cut-away side elevational view of an intravascular electrical lead suitable for use as the heart activity sensing and therapy delivering element of an automatic cardioverting device;

FIG. 3 is a detailed electrical schematic diagram of a heart function monitoring circuit suitable for use as the monitoring means of FIG. 1;

FIG. 4 is an exemplary electrical schematic diagram of a circuit suitable for use as the delay and patient alert blocks in the diagram of FIG. 1;

FIGS. 5A through 5D are electrical schematic diagrams of alternative patient interrupt means suitable for use in combination with the circuit of FIG. 3 and as represented in the block diagram of FIG. 1;

FIGS. 6A through 6D are further alternative patient interrupt circuits for cases where it is desired that periodic actions by the patient for continued inhibiting of the operation of the automatic therapy means be included; and

FIG. 7 is an electrical schematic circuit diagram of a therapy circuit for delivering cardioverting pulses to the heart, as represented in the block diagram of FIG. 1.

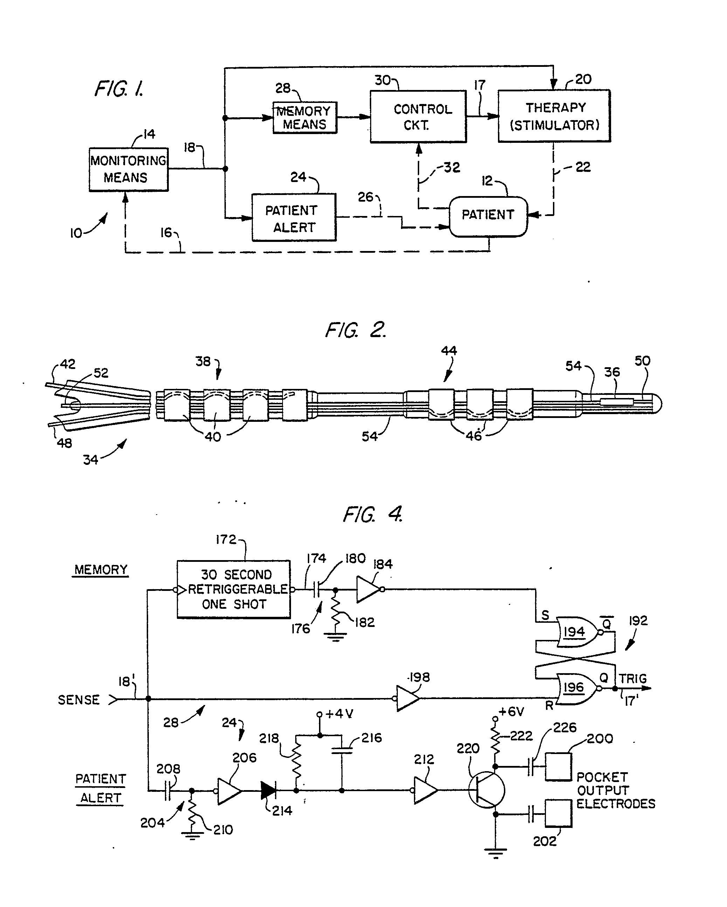

Referring first to FIG. 1, there is shown a general block diagram illustrating general concepts of the present device. FIG. 1 is primarily a conceptual illustration and is not intended to indicate a particular way in which various hardware elements are interconnected in an actual implementation, although a hardware implementation along the lines of FIG. 1 is, of course, possible. In FIG. 1, the rectangular blocks represent elements of a body-implantable therapy device, generally designated 10, which is adapted to be implanted within the body of a patient 12. The solid lines represent the paths of signals between the various elements of the therapy device 10, and the dash lines represent communications between the therapy device 10 and the patient 12.

More particularly, the therapy device 10 comprises a body function monitoring means 14 which monitors for the occurrence or existence of a condition within the body of the patient 12 which would require therapy. In the particular embodiment described herein in detail, the body function monitoring means 14 monitors heart function for the existence of a life-threatening arrhythmic condition, for example ventricular defibrillation. The actual monitoring is done through a connection represented by the dash line 16 extending from the patient 12 to the monitoring means 14.

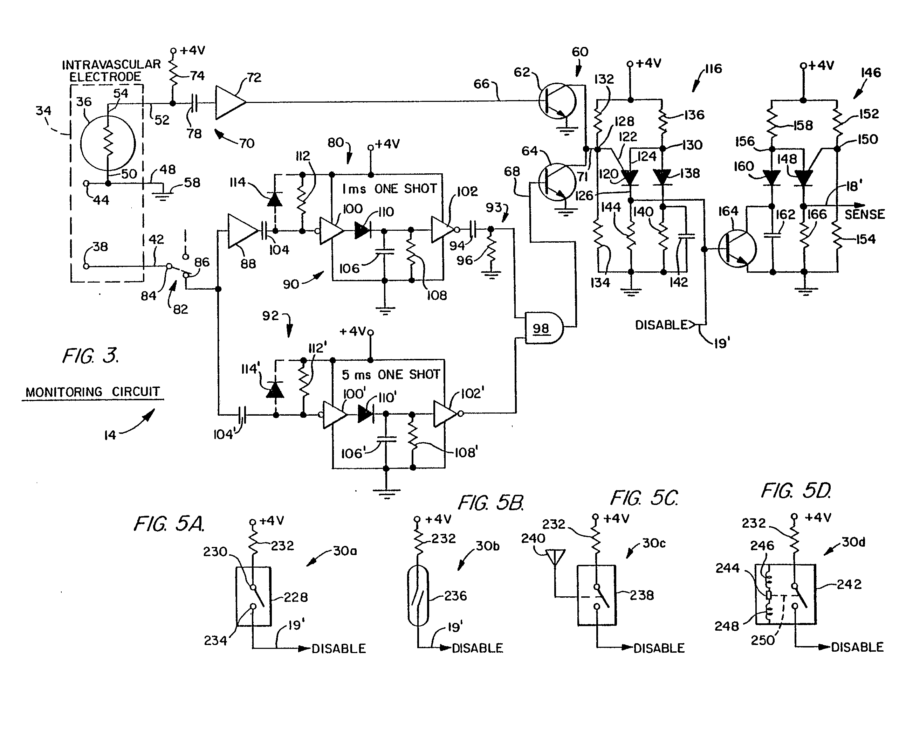

The body function monitoring means 14 generates a signal along a line 18 when the condition requiring therapy apparently exists. As previously mentioned, the condition requiring therapy may actually be present and thus a signal on the line 18 would be genuine, or a signal on the line 18 may be false as a result of external interference with the monitoring means 14 or other malfunction associated therewith. One example of a suitable circuit where the monitoring means 14 is a heart function monitoring means for generating a signal indicating the apparent existence of a life-threatening arrhythmic condition is described below in detail with particular reference to FIG. 3.

Still referring to FIG. 1, the therapy device 10 also includes a triggerable means 20 for administering therapy appropriate for the conditon which the monitoring means 14 is intended to sense. The therapy is administered to the patient through a means represented by a dash line 22. In the case where the monitoring means 14 monitors for a life-threatening arrhythmic heart condition, appropriate therapy is defibrillation, and the therapy means 20 would be a suitable cardioversion means or heart stimulator. A suitable cardioversion therapy circuit is descirbed in detail below with particular reference to FIG. 7.

In accordance with the invention, when the monitoring means 14 indicates the apparent existence of the condition requiring therapy, the patient 12 is alerted and given the option to determine what course of action the therapy devide 10 should take, and more particularly, whether the therapy means 20 should be triggered. To this end, the signal line 18 from the monitoring means 14 is connected to a patient alert means 24 which, when activated, alerts the patient 12 through a means generally represented by a dash line 26. One suitable example of such a patient alert means is described below with particular reference to FIG. 4. The signal line 18 is also connected to the therapy means 20 to initiate or to prepare the therapy means 20 to effect its therapy operation at a point later in time after the memory means 28 has timed out. In the embodiment where the therapy means 20 takes the form of a defibrillator, which embodiment will be described with respect to Figure 7, the signal applied to the therapy means 20 initiates or causes the capacitor of the defibrillator to charge or to maintain its charge as long as the monitoring means 14 develops a SENSE signal upon line 18. In known defibrillators, there is included a capacitor which is charged to a relatively high voltage. Thus, it is not desired to maintain the defibrillator's capacitor charged continuously; but, as indicated, the SENSE signal developed by the monitoring means 14 is used as a preparatory signal to initiate the defibrillator's capacitor to be charged. In other contemplated embodiments of this invention, the therapy means 20 may perform other types of therapy operations upon body tissue, and the SENSE signal is applied to the therapy means 20 as a preparatory signal to prepare the therapy means for operation in response to a signal applied later thereto by the patient control circuit 30 to actually initiate its operation.

The signal output line 18 from the monitoring means 14 is additionally connected to a memory means 28 which serve to permit the therapy means 20 to effect therapy a predetermined time after the monitoring means 14 generates a signal. The patient 12 has the predetermined time in which to take appropriate action. In the particular configuration illustrated in FIG. 1, a patient control circuit 30 receives a communication from the patient 12 through a means generally represented by a dash line 32 and operates to control the operation of the therapy means 20. In the particular configuration illustrated, the patient control circuit 30 is, for clarity, shown interposed between the memory means 28 and the therapy means 20. However, FIG. 1 is a conceptual illustration only, and it will be apparent that there are numerous ways in which operation of the therapy means 20 may be controlled, other than by controlling passage of a signal from the memory means 28 to the therapy means 20.

In the operation of the therapy device 10, as depicted in FIG. 1, when the monitoring means 14 generates a signal on the line 18 indicating the apparent existence of a condition requiring therapy, for example, a life-threatening arrhythmic heart condition, the patient alert means 24 alerts the patient 12 who may then determine what course of action the device 10 should take, and specifically, whether the therapy means 20 should be triggered.

In a more particular mode of operation, the therapy means 20 may be actuated during the period established by memory means 28, i.e., a predetermined time period after the monitoring means 14 generates a signal. During this period, the patient may manipulate the control circuit 30 to apply a TRIG signal via line 17 to the therapy means 20, thus administering therapy to the patient. It is contemplated within the teachings of this invention that the patient control circuit 30 may operate in at least two different modes. In a first mode, the patient 12 must take a defined action in order to enable the patient control circuit 30 to permit the therapy means 20 to be actuated. In this first mode, the patient, upon sensing the stimulation or manifestation of the patient alert 24, manipulates the patient control circuit 30 in a manner to be explained, whereby the therapy means 20 will then apply an appropriate therapy to the patient 12. In the first mode, the memory means serves to store the therapy condition signals derived from the monitor means for a predetermined time during which the patient may operate his control means 30 to effect the administering of therapy. In a second or interrupt mode, the patient control circuit 30 takes the form of an interrupt means, which the patient 12 actuates only if he desires to prevent or interrupt the actuation of the therapy means 20. The interrupt mode is of particular interest if the therapy means 20 illustratively takes the form of a defibrillator. If the defibrillation is in fact occurring and the signal on the line 19 is therefore genuine, then the patient, likely unconscious, need take no action and the therapy automatically occurs. If on the other hand the signal from the monitoring mode 14 on the line 18 is a result of a false response or other malfunction, then the patient, upon being alerted by the patient alert 24, and feeling no heart pain, recognizes that therapy would be inappropriate. Accordingly, the patient 12, by means of the patient control means 30, inhibits operation of the therapy means 20. In the inhibit mode of operation, the memory means is in effect a delay means for delaying the application of the therapy condition signal for a period of time before applying it to the therapy means, so that the patient may take action by operating the control means to inhibit the activation of the therapy means.

If for some reason, which may or may not be the result of a malfunction, the monitoring means 14 and the patient alert 24 continuously operate while the patient 12 perceives there is no need for therapy, the patient 12 should immediately seek medical attention, and of course at the same time operate the patient control circuit 30 to inhibit the therapy means 20. However, the patient 12 may instead view such continuous operation of the patient alert means 24 as a mere nuisance and seek to avoid the supposed nuisance by continuously operating the patient control circuit 30, for example by taping a magnet to his chest where the patient control circuit 30 includes a magnetically operable switch. To prevent the patient 12 from so avoiding such a supposed nuisance, the patient control circuit 30 in its interrupt embodiment or mode is preferably designed to require periodic positive actions by the patient for continued inhibiting of the operation of the therapy means 20. In such a case, by means of a connection not shown in FIG. 1, the patient 12 is alerted by the patient alert means 24 when another positive action is required in order to continue the inhibiting mode of the operation of the therapy means 20.

While it will be apparent that the inventive concept illustrated in FIG. 1 is particularly applicable to an automatic heart defibrillator, it will be apparent that other applications are possible. Examples are an automatic insulin dispensor or a demand pacemaker where the patient 12 has the option of overriding a preset heart rate limit. In the example where the device is adapted to dispense insulin, the patient could override the operation of the therapy device by actuating the patient control circuit 30 if he knew that the monitoring means 14 had sensed a dietary abnormal condition, for example the eating of a candy bar. In another example, the body-implantable therapy device could take the form of a physiological responding pacemaker, which acts to increase the rate of breathing or effects a blood chemistry change. In such a device, the monitoring means taking the form of a respiration sensor may inadvertently respond to the patient's hiccups. Accordingly, it will be appreciated that the body-implantable therapy device 10 described below as an automatic cardioverting device, more particularly an automatic defibrillator, is by way of example only.

Referring now to FIG. 2, to serve the dual functions of detecting normal heart activity (represented by the dash line 16 in FIG. 1) and providing a means for introducing an electrical cardioverting pulse into the heart (represented by the dash line 22 in FIG. 1), an intravascular electrode 34 is provided. The particular intravascular electrode 34 illustrated is described in greater detail in US—A—3,815,611, the entire disclosure of which is hereby incorporated by reference. Briefly, the intravascular electrode 34 redundantly monitors for normal heart activity by means of separate contraction and EKG sensors. As is more particularly described in US—A—3 815 611 the contraction sensor is a variable resistance 36 comprising a vinyl elastomer loaded with fine particles of carbon black such that its resistance is a function of the density of the carbon particles, which in turn depends upon the degree of bending of the variable resistance 36. In order to sense heart electrical activity, specifically to sense an EKG, a proximal electrode 38 comprising a plurality of separate conductive rings 40 is provided. A conductor 42 makes electrical connection with the proximal electrode 38. In addition to sensing heart electrical activity, the proximal electrode 38 also serves to introduce an electrical cardioverting pulse into the patient's heart when appropriate.

To provide a common (ground reference) return for the proximal electrode 38, a distal electrode 44 is included, which similarly comprises a plurality of spaced conducting rings 46. A common lead 48 is connected to the distal electrode 44, and additionally to the far end 50 of the contraction sensor 36.

To complete the circuit to the contraction sensor 36, a third electrical lead 52 is connected directly to the near end 54 of the contraction sensor 36.

As is more particularly described in the above-incorporated US—A—3 815 611, the entire intravascular electrode 34 is flexible, and the body 54 thereof preferably comprises a flexible silicone rubber casting compatible with body fluids.

Referring now to FIG. 3, there is shown an illustrative example of an electrical schematic diagram of the monitoring circuit 14, as generally shown in FIG. 1. The monitoring circuit 14 is substantially identical to one which is disclosed in the above-incorporated, US—A—3,805,795. Accordingly, the monitoring circuit 14 is only briefly described herein, and reference may be had to US—A—3,805,795 for a further description.

Preliminarily, it should be noted that the circuitry described herein with reference to FIG. 3, as well as to FIGS. 4—7, includes a suitable power supply, which herein is represented by +4 volt and +6 volt source terminals. As is more particularly described in the Denniston et al U.S. Patent 3,805,795, a suitable power supply may comprise a 6-volt battery and a 4-volt voltage regulator.

The intravascular electrode 34 to FIG. 2 will be seen to comprise the input element to the circuit 14 of FIG. 3. The circuit 14, through the intravascular electrode 34, monitors two dynamic characteristics of a normally beating heart. The absence of both of these characteristics for a predetermined period of time indicates a lack of normal heart function, and the circuit 14 serves to output a logic high signal on a SENSE line to indicate the apparent existence of a life threatening arrhythmic heart condition.

More specifically, the monitoring circuit 14 monitors EKG by means of the electrodes 38 and 44 of the intravascular electrode 34, and muscle contraction by means of the contraction sensor 36 of the intravascular electrode 34. As shown in FIG. 3, the common lead 48 is connected to a circuit ground 58 in order to conveniently serve the various functions of the intravascular electrode 34.

In order to indicate a normally functioning heart if either one of the dynamic characteristics of the heart is present, the monitoring circuit 14 includes an OR gate 60 comprising a pair of NPN transistors 62 and 64 connected in common emitter configuration with their collector electrodes tied together. A line 66 connected to the base of the upper transistor 62 periodically goes high in response to normal heart muscle contractions, and a line 68 connected to the base of the lower transistor 64 periodically goes high in response to normal EKG signals. Thus, at least one of the transistors 62 or 64 periodically conducts in response to normal heart function, pulling a shared collector output line 71 low.

For sensing normal heart contraction, a contraction sensor circuit 70 comprises an amplifier 72 with signals from the contraction sensor 36 applied to the input thereof. More specifically, a biasing resistor 74 is connected between a +4 volt source and the lead 52 from the contraction sensor 36. To couple changes in current flowing through the resistor 74 and the sensor 36, a capacitor 78 is connected between the junction of the resistor 74 and the contraction sensor 36 to the input of the operational amplifier 72. The output of the operational amplifier 72 is connected to the line 66.

In the operation of the overall contraction sensor, as the contraction sensor resistance 36 flexes as a result of normal heart activity, it changes resistance and electrical pulses are coupled through the capacitor 78 and amplified by the operational amplifier 72. The line 66 periodically goes high, biasing the transistor 62 into conduction to indicate normal heart activity.

An EKG sensor 80 serves to amplify each R-wave signal detected by the electrode 38 of the intravascular electrode 34 corresponding to a normal heartbeat. More specifically, EKG sensor 80 amplifies R-waves produced by a human heartbeat, discriminating against electrical heart waves produced by a heart in fibrillation or otherwise abnormally functioning, as well as discriminating against pacer (pacemaker) pulses which may be applied through the intravascular electrode 34.

The EKG sensor 80 is connected to the electrode 38 through the lead 42, and through contacts of a reed relay 82 shown in phantom lines in FIG. 3. At this point in the description, it is sufficient to note that electrical connection is made from the electrode 38 to the EKG sensor 80 by means of a common contact terminal 84 and a normally closed contact terminal 86 of the reed relay 82.

The terminal 86 is connected to the input of a suitable R-wave amplifier 88 which, in turn, drives a compensated monostable multivibrator 90. In one particular embodiment, the multivibrator 90 has a triggering threshold of 10 mv, and an output pulse width of 1 ms.

The terminal 86 is also directly connected to the input of another compensated monostable multivibrator 92 which, in a preferred embodiment, has an input triggering threshold of 0.5 v and a pulse width of 5 ms.

At the output of the multivibrator 90 is a differentiating network 93 comprising a series capacitor 94 and a resistor 96 connected between the output end of the capacitor 94 and circuit ground.

The output of the differentiating network 93 of the monostable multivibrator 90 and the output of the monostable multivibrator 92 are connected to the inputs of an AND gate 98 which is activated if, at the time it receives a logic high pulse from the differentiating network 93, the output of the multivibrator 92 is also high. If, at the time a logic high pulse is received from the differentiating circuit 93, the output of the multivibrator is low, then the AND gate 98 is not activated, and its output remains low.

Many suitable circuits are possible for the compensated monostable multivibrators 90 and 92. Since these two monostable multivibrators 90 and 92 are identical except for the particular component values which establish the input thresholds and timings, only the upper monostable multivibrator 90 is described below, with corresponding elements of the lower monostable multivibrator 92 being designated by primed reference numerals.

The multivibrator 90 comprises a pair of inverters 100 and 102 which comprise NAND gates with the inputs tied together. An input capacitor 104 is provided to couple low going pulses to the inverter 100. A storage and discharge network comprising a capacitor 106 and a resistor 108 is connected between the input of the inverter 102 and ground, and is supplied through an isolation diode 110 from the output of the inverter 100. A pull-up resistor 112 connects the junction of the input capacitor 104 and the input of the inverter 100 to the +4 volt source. A diode 114 in parallel with the resistor 112 is part of the input protection circuit of the inverter 100.

In the operation of the compensated monostable multivibrator 90, the multivibrator 90 is triggered by a low going pulse applied to the capacitor 104 and responds with a low going pulse of predetermined duration at the output of the inverter 102. More particularly, in the quiescent state, the input of the inverter 100 is high and the output low. The input of the inverter 102 is therefore low, and its output high. When a negative going pulse is introduced through the capacitor 104 from the R-wave amplifier 88 (or directly from the electrode 38 through the capacitor 104' in the case of the lower monostable multivibrator 92), this pulse is coupled through the capacitor 104 to the input of the inverter 100, and the output of the inverter 100 goes high. The capacitor 106 then quickly charges through the diode 110 and the inverter 100 to +4 volts, and the output of the inverter 102 goes low, generating the beginning of the output pulse. As the capacitor 104 discharges negatively, it charges through the resistor 112 to +4 volts. The output of the inverter 100 remains high until the voltage waveform generated by the charging of the capacitor 104 passes through the transfer voltage of the inverter 100, whereupon its output goes low. The isolation diode 110 temporarily prevents the discharge of the capacitor 106, which then commences to discharge to ground through the resistor 108. The output of the inverter 102 remains low, thus continuing the output pulse until the waveform generated by the discharge of the capacitor 106 passes through the transfer voltage point of the inverter 102, whereupon its output goes high thus ending the output pulse.

The end of the output pulse of the upper monostable multivibrator 90 is detected by the differentiating network 93 which couples a logic high spike to the AND gate 98 which is activated if it is enabled by the output of the lower multivibrator 92.

The overall operation of the EKG sensor 80 is based upon R-waves from a human heart beating in normal sinus rhythm having a magnitude in the 20 mv range when sensed through the intravascular electrode 34 system. Pacer pulses are commonly in the 1.0 to 2.0 voltage range. Accordingly, a normally produced R-wave is insuffient to trigger the lower multivibrator 92 into its logic low output stage. However, the output of the R-wave amplifier 88 is sufficient to trigger the upper multivibrator 90 into its logic low output state, and thus causes the differentiating network 93 to supply a logic high spike pulse to the AND gate 98 when the output of the multivibrator 90 returns high 1 ms later. The AND gate 98 is momentarily activated, and the output line 68 goes high to bias the NPN transistor 64 into conduction, thus pulling the output line 71 low.

Conversely, pacer pulses trigger both multivibrators 90 and 92. Since the output of the upper multivibrator 90 is effectively delayed 1 ms (and inverted) by the differentiating network 92, and the logic low output pulse of the monostable multivibrator 92 is 5 ms in duration, the output of the monostable multivibrator 92 is low when the AND gate 98 receives the positive pulse from the multivibrator 90, and the AND gate 98 is accordingly not activated.

Receiving the output of the OR gate 60 along the line 71 is a pulse shaper 116 which serves to shape low going pulses along the line 71 into high going pulses of substantially the same pulse width and amplitude along an output line 118.

Specifically, the pulse shaper 116 comprises a programmable unijunction transistor (PUT) 120 electrically connected in a monostable multivibrator arrangement. The PUT has gate 122, anode 124, and cathode 126 electrodes. The PUT 120 is rendered conductive, thereby providing a low impedance from both the gate 122 and anode 124 to the cathode 126, when the anode voltage exceeds the gate voltage by a specified amount, for example 0.7 volt.

The gate and anode terminals 122 and 124 are respectively connected to nodes 128 and 130 of a pair of voltage dividers connected between the +4 voltage source and ground. The node 128 is a tap point of a first voltage divider comprising resistors 132 and 134, and the node 130 is a tap point of a second voltage divider comprising a resistor 136, a diode 138, and another resistor 140 shunted by a capacitor 142. To complete the pulse shaper circuit 116, a load resistor 144 is connected between the cathode terminal 126 and circuit ground, with the output line 118 also connected to the cathode terminal 126. The component values in the circuit 116 are such that the PUT 120 is normally non-conducting. That is, the voltage at the node 130 is less than 0.7 volts lower than the voltage at the node 128.

In the operation of the pulse shaper 116, whenever the OR gate 60 is activated, the line 71 goes low, providing a low resistance path between the node 128 and ground, shunting the resistor 134. The voltage on the PUT gate 122 is now lower than the voltage of the anode 124, and the PUT 120 accordingly conducts. Conduction of the PUT 120 pulls the output line high through the resistors 132 and 136. The isolation diode 138 prevents the capacitor 142 from discharging through the PUT 120, and accordingly the capacitor discharges through the resistor 140. The component values of the capacitor 142 and the resistor 140 are selected such that the capacitor 142 discharges at a rate predetermined to keep the voltage at the node 130 sufficiently high to keep the PUT 120 conductive for a predetermined period of time. This time period establishes the pulse width output on the line. When the capacitor 142 is discharged, the anode 124 voltage drops to less than 0.7 volt above the gate 122 voltage, and the PUT 120 ceases conducting.

A control circuit 146 receives the output of the pulse shaper 116. The control circuit 146 is a timing and latch circuit which causes the output SENSE line 18' to go high when pulses have not been received from the pulse shaper 116 for five seconds, and to hold the SENSE line high so long as no pulses are received from the pulse shaper 116. The control circuit 146 comprises another PUT 148 having its gate terminal connected to a node 150 at a tap point of a voltage divider comprising resistors 152 and 154, and its anode terminal connected to a node 156 in a voltage divider comprising a resistor 158, an isolation diode 160, and a capacitor 162 shunted by the collector and emitter terminals of an NPN transistor 164. To complete the control circuit 146, an output load resistor 166 is connected between the cathode terminal of the PUT 148 and circuit ground, with the SENSE line also connected to the PUT cathode terminal.

In the operation of the control circuit 146, the PUT 148 is normally non-conducting, and the voltage at the node 150 connected to the gate terminal remains constant until such time as the PUT 148 is triggered into conduction. However, the voltage on the node 156 varies depending upon the charge on the capacitor 162, which charge varies as the capacitor 162 is charged from the +4 volt source through the resistor 158 and the isolation diode 160, and is periodically discharged by the NPN transistor 164 as the input line periodically goes high. In a preferred embodiment, the component values of the capacitor 162, the diode 160, and the resistor 158 are chosen such that it takes approximately 5 seconds to charge the capacitor 162 to a voltage which renders the PUT 148 conductive. The diode 160 prevents the capacitor 162 from discharging through the PUT 148. Pulses from the pulse shaper 116 are of sufficient duration to totally discharge the capacitor 162 in response to each heartbeat.

If not, pulses are passed through the OR gate 60 and the pulse shaper 116 for 5 seconds or longer, the PUT 148 becomes conductive, thus pulling the output SENSE line 18' high. The SENSE line 18' remains high for as long as no further input pulses are received along the input line, and the control circuit 146 is not otherwise reset.

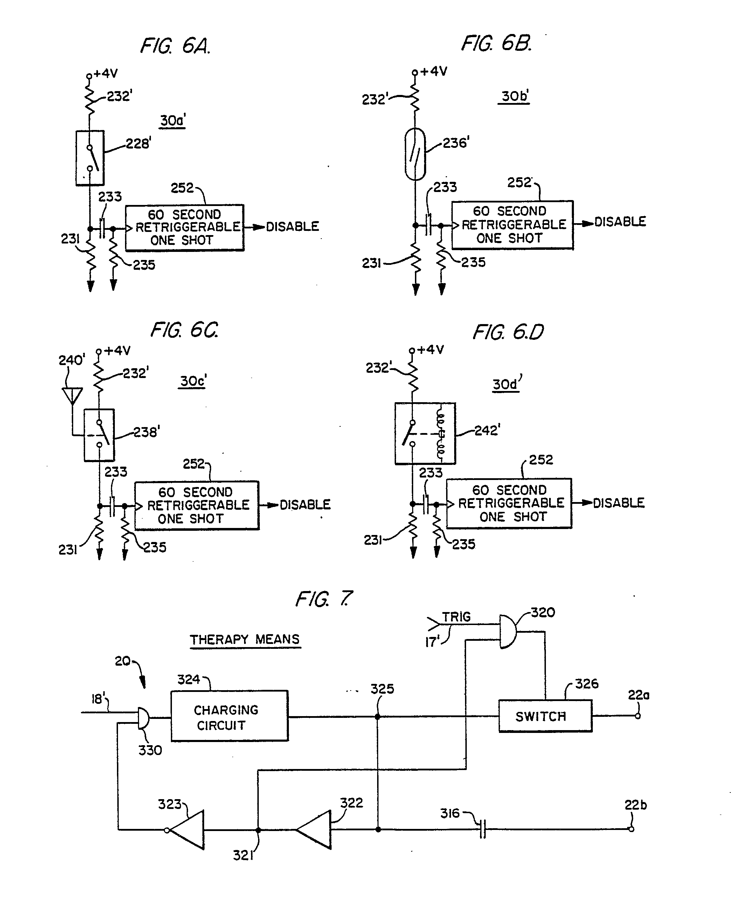

In FIG. 3, it will be seen that the control circuit 146 receives a DISABLE signal via line 19' coupled to the transistor 164, which will be further described below with particular reference to FIGS. 5A through 5D and 6A through 6D. The architecture of the specific embodiment as described with respect to FIGS. 3, 4, 5, 6, and 7 is slightly different from that generally and theoretically shown in FIG. 1. To identify these differences, the lines interconnecting the circuits of the specific embodiment are noted by primes, for example, lines 17', 18', and 19'. As will be more fully explained, line 18' interconnects the control circuit 146 of the monitoring circuit 14 directly to the therapy means 20 as shown in FIG. 7; each of the control circuits as shown in FIGS. 5 and 6 is connected by a line 19' to the DISABLE input of the monitoring circuit 14 as shown in FIG. 3; and the TRIG signal as developed by the memory means 24 as shown in FIG. 4, is applied by a line 17' to the therapy means 20, as shown in FIG. 7.

Referring now to FIG. 4, there is shown detailed circuit diagrams of an illustrative embodiment of each of the memory means 28 and patient alert circuit 24, shown generally in FIG. 1. Both the memory means 28 and the patient alert circuit 24 of FIG. 4 has as inputs the SENSE line 18' from the control circuit 146 of FIG. 3. SENSE goes high whenever a heartbeat has not been detected for at least 5 seconds, thus indicating the apparent existence of an arrhythmic heart condition.

The memory means 28 serves to output a logic high signal on a TRIG line 17' seconds following the SENSE signal. The TRIG line is used to trigger the therapy circuit as represented generally by the block 20 of FIG. 1, and more particularly shown in FIG. 7 described below.

More particularly in FIG. 4, the memory means 28 comprises a 30 second retriggerable one shot 172 which is triggered on a low to high transition on the SENSE line 18'. The embodiment of the memory means 28 illustrated in FIG. 4 is particularly adapted for operation in the second or delay mode, providing a delay before generating its high signal on the TRIG line. The retriggerable one shot 172 is a conventional one, and generates a pulse at its output 174 each time it is activated. In this particular circuit the retriggerable one shot 172 has a low active output, and thus the output 174 goes low for a predetermined time each time the input is triggered by a low to high transition on the SENSE line. In this particular embodiment, a 30 second time delay is provided by the retriggerable one shot 172, and thus the output 174 remains low for 30 seconds before transitioning to high, unless further low to high transitions occur on the SENSE line 18' retriggering the one shot 172. The above-described embodiment of the memory means 28 as shown in FIG. 4 contemplates a device that imparts a delay to the trigger signal before being applied to the patient control circuit 30, permitting the patient to interrupt the application of an enabling signal to the therapy means 20. When the patient control circuit 30 is operative in its first or control mode, the memory means 28 functions as a memory to retain the occurrence of the detection by the monitoring means 14 of a condition requiring therapy for a period of time to permit the patient to actuate the patient control circuit 30 in a direct positive action. In this mode, the memory means 28 may illustratively take the form of a retriggerable one shot similar to the element designated 172, which would provide an output signal for a desired period of time, e.g., 30 seconds.

A differentiating network 176, comprising a series capacitor 180 and a resistor 182 connected to ground, serves to pass a high going pulse to a buffer 184 at the end of the one shot 172 output pulse when the output 174 transitions from low back to high. The memory means 28 also has a set-reset flip-flop 192 comprising a pair of crosscoupled NOR gates 194 and 196. As is conventional, the flip-flop 192 has set (S) and reset (R) inputs and $Q$ and $\overline{Q}$ outputs. The $Q$ output taken from the output of the NOR gate 196 applies the TRIG signal to the line 17'. The output of the buffer 184 is connected to the set (S) input, and the SENSE line is connected through an inverter 198 to the flip-flop 192 reset (R) input.

In the operation of the memory means 28 of FIG. 4 in its delay mode, SENSE is normally low and the inverter 198 is activated, thus supplying a logic high to the reset (R) input of the flip-flop 192, activating the lower NOR gate 196. Thus the $Q$ output which supplies the TRIG line 17' is low. At the same time, the buffer 184 supplying the flip-flop set (S) input is inactive, and both inputs of the upper NOR gate 194 are low. The upper NOR gate 194 is therefore inactivated, and $\overline{Q}$ is high. When SENSE goes high, indicating the apparent existence of an arrhythmic heart condition, the output of the inverter 198 goes low, thus removing the reset input from the flip-flop 192. However, the flip-flop 192 does not change state at this point because the logic high $\overline{Q}$ output connected back to the input of the NOR gate 196 keeps the NOR gate 196 activated, and $Q$ and TRIG line 17' remains low. The SENSE line transitioning from low to high also triggers the 30-second retriggerable one shot 172, and its output 174 goes low. Since the buffer 184 is inactivated at this point because its input is pulled low anyway by the resistor 182, the low going input pulse has no additional effect. At the end of the output pulse from the one shot 172, the output 174 goes high and this high going pulse is coupled through the capacitor 180 to activate the inverter 184, which activates the NOR gate 194, and $\overline{Q}$ goes low. With $\overline{Q}$ low, the NOR gate 196 is no longer activated, and $Q$ as well as TRIG go high. Thus the therapy means 20 (FIG. 1) is triggered.

In the memory means 28 of FIG. 4, since the TRIG output signal is taken from the lower NOR gate 196 which receives the reset (R) input which, in turn, is supplied through the inverter 198 directly from the SENSE line 18', it will be apparent that if SENSE returns low at any time, the inverter 198 and the NOR gate 196 are immediately again activated, thus immediately terminating the TRIG output signal. There are a number of circumstances under which the SENSE line 18' might return low after an initial high condition. Examples are a normal heartbeat again being sensed, stopping of the defibrillator as a result of a signal being produced from the therapy circuit of FIG. 7, or in the particular context of the second mode of operation, intervention by the patient.

The patient alert circuit 24 of FIG. 4 has as its output element a pair of pocket output electrodes 200 and 202 which are mounted to the outside of the case of the implanted device so as to stimulate the tissue surrounding the device when an electrical voltage is impressed thereacross. The patient alert circuit 24 performs this function in response to a low to high transition on the SENSE line 18'. It should be noted that the patient alert circuit 24 is described in greater detail in the above-mentioned, US—A—4,140,131, the entire disclosure of which is hereby incorporated by reference.

Specifically, the patient alert circuit 24 comprises an input differentiating network 204 connected to the input of an inverter 206. The differentiating network 204 includes a capacitor 208 connected in series with the input of the inverter 206, and a resistor 210 connected between the inverter input and circuit ground. The output of the inverter 206 is connected to the input of another inverter 212 through an isolation diode 214 and a pulse stretching network comprising a capacitor 216 and a resistor 218 connected in parallel between the input of the inverter 212 and the +4 volt source. The output of the inverter 212 is connected to the base of an NPN transistor 220 connected in common emitter configuration, with its collector connected through a load resistor 222 to a +6 volt source. To complete the circuit 24, the collector of the transistor 220 is connected through coupling capacitor 226 to the pocket output electrode 200, and the grounded emitter is connected to the pocket output electrode 202.

In the operation of the patient alert circuit 24, a low to high transition on the SENSE line is coupled through the capacitor 208 to momentarily activate the inverter 206. The pulse stretching capacitor 216 discharges through the isolation diode 214 and the inverter 206, activating the inverter 212, the output of which biases the transistor 220 into conduction. A low going output pulse is coupled through the capacitor 226 to the pocket output electrode 200, thus stimulating the tissue surrounding the implanted automatic defibrillator. This tissue stimulation is felt by the patient 12, who thereby is advised that if he takes no action a defibrillating pulse will be delivered to his heart shortly after the 30 second output pulse from the retriggerable one shot 172 ends.

While this illustrative circuit 24 delivers only a single tissue stimulating pulse to be felt by the patient 12, a continuous or periodic stimulation may readily be provided if desired by substituting for the particular circuit 24 an oscillator (not shown) to continuously or periodically pulse the transistor 220 whenever SENSE is high.

Referring now to FIG. 5A, there is shown an illustrative embodiment of a patient control circuit 30a, as represented by the block 30 of FIG. 1, suitable for use in combination with the circuits of FIGS. 3 and 4 described above. Specifically, the patient control circuit 30a operates in a delay mode and comprises a switching element, generally represented by a switch 228, which is operable by the patient 12 for inhibiting operation of the therapy means 20. While the representative switch 228 comprises a conventional switch contact, it will be apparent that numerous forms of switching elements are possible, including solid state switching elements such as transistors. In the particular circuits herein described, the upper terminal 230 of the representative switch 228 is connected through a current limiting resistor 232 to the +4 volt supply. The lower switch terminal 234 is connected to supply the DISABLE line which is connected to the DISABLE input of the control circuit 146 of the FIG. 3 monitoring means 14. Accordingly, whenever the switch 228 is closed, DISABLE goes high, biasing the transistor 164 (FIG. 3) into conduction, discharging the capacitor 162, and causing the PUT 148 to become and remain non-conducting. The SENSE pulse immediately terminates, thus resetting the flip-flop 192 of FIG. 4, and ending any TRIG pulse. A DISABLE signal being produced before the expiration of the 30 second pulse from the one shot 172, causes SENSE to go low, thus insuring that the flip-flop 192 Q output remains low (by holding the reset (R) input high), even though a momentary pulse is later applied to the (S) input upon the expiration of the 30 second period.

As mentioned above, in FIG. 5A a generalized representation of the switching element 228 is depicted. FIGS. 5B, 5C, and 5D depict suitable exemplary specific switches which might be employed.

In FIG. 5B, a control means 30b is illustrated as a magnetically actuated reed switch 236. In the presence of a magnetic field, the contacts of the reed switch 236 close, and DISABLE goes high.

Accordingly, the patient 12 may simply carry a magnet to be placed over the implanted device location when it is desired to disable the therapy means 20.

In FIG. 5C, a control means 30c is illustrated as an electromagnetic field responsive device 238, for example a radio receiver, which receives the command from the patient 12 to inhibit the therapy means 20. The receiver 238 has as its input element an antenna 240 to receive the electromagnetic energy. In actual practice, the entire electromagnetic field responsive device 238 may comprise little more than the antenna 240 which is tuned, in a known manner, to receive pulses or bursts of radio frequency energy on a predetermined carrier frequency, for example, 175 kHz. The use of such antennas having a frequency response limit to particular frequency ranges is well known in the art and serves to minimize undesired response of the unit to extraneous noise. A suitable electromagnetic field responsive device is described in the above-mentioned US—A—4,066,086, the entire disclosure of which is hereby incorporated by reference.

As a third actual embodiment, FIG. 5D illustrates a control means 30c as a mechanical shock sensor 242. The mechanical shock sensor 242 is an accelerometer type device as is represented by the mass 244 suspended by two springs 246 and 248 and operatively connected as represented by the dash line 250 to close the switch contacts when a sufficient mechanical shock is detected. Thus all the patient need do is vigorously thump his chest in the vicinity of the pacemaker in order to disable the therapy means 20. Though it is contemplated that a variety of mechanical shock sensors 232 could be incorporated as the control means 30c, in an illustrative embodiment of this invention, a mechanical shock sensor as taught by one of the following patents could be incorporated; US—A—3,916,173; US—A—2,897,306; and US—A—2,793,260; of the aforenoted sensors, the sensor described in US—A—3 946 173 would be preferred.

One disadvantage associated with the use of the form of patient control means illustrated in FIGS. 5A through 5D is that, particularly in the case of the magnetic field responsive switch 236 of FIG. 5B, the patient might attempt to defeat the device 10 by permanently providing a patient interrupt actuating signal, for example by taping a magnet to his chest over the implanted defibrillator. As mentioned above, a patient might do this to avoid what he perceives to be a nuisance alarm, thus defeating the purpose of the entire device 10. If the alarm is in fact a false alarm, then the patient should immediately have the malfunction corrected, rather than effectively disabling the device 10 for an indeterminate period of time.

To render such an attempt ineffective, the circuits of FIGS. 6A through 6D may be employed. FIGS. 6A through 6D are identical to corresponding FIGS. 5A through 5D, with the addition in each case that a 60 second retriggerable one shot 252 is interposed between the lower switch terminals and the DISABLE line. The identical switch elements in each case are designated by primed reference numerals. The output of the switch elements is connected across a resistor 231 to ground. The output signal established across the resistor 231, being applied to a differentiating circuit comprised of a capacitor 233 coupled to the input of the 60 second, retriggerable one shot 252, and a resistor 235 coupled to ground. The differentiating circuit output provides a leading edge to retrigger the one shot 252. The function of the retriggerable one shot 252 is to insure that periodic positive actions by the patient 12 are required for continued inhibiting of the operation of the therapy means 20. The one shot 252 is of the type which is triggered on the leading edge of a high going logic transition as provided by the differentiating circuit.

In the operation of the circuits shown, for example that of FIG. 6B, upon closure of the reed switch contacts 236' by an externally applied magnetic field, the voltage developed across the resistor 231 is differentiated by the R—C circuit to provide a signal with a leading edge to trigger the one shot 252, whereby its output goes high and the DISABLE line goes high. Thus the therapy means 20 is inhibited. At the end of the 60 second pulse produced by the one shot 252, DISABLE again goes low and remains low until such time as the one shot 252 is retriggered by another closure of the reed switch 236'. Since the retriggerable one shot 252 requires a transition on its input, if the reed relay contact 236' remains closed by virtue of the magnet being permanently held in position, no such retriggering occurs.

In a similar manner, the embodiments of FIGS. 6A, 6C, and 6D all serve to insure that periodic positive actions by the patient are required for continued inhibiting of the operation of the therapy means 20.

Referring lastly to FIG. 7, an illustrative therapy circuit 20 suitable for use as the therapy means 20 of FIG. 1 is shown. The therapy circuit 20, when activated by the TRIG signal applied by the memory means 28 to line 17', functions to supply electrical cardioverting pulses to the patient's heart at 1000 volts through the terminals 22a and b, and the proximal electrode 38 of the intravascular electrode 34 (FIG. 3).

With reference to FIG. 7, there is shown an illustrative embodiment of the therapy means 20 of FIG. 1. The line 17' applies the TRIG signal to the therapy means and serves as one input to an AND gate 320. The other input to AND gate 320 is connected to a junction 321 which is connected to the output of a comparator 322. Comparator 322 is of a type known to the prior art having an internal reference level against which signals appearing at its input are compared, the output of comparator 322, and thus junction 321, being high if the input to comparator 322 equals or exceeds the reference level and low if the input is below the reference level. Junction 321 is connected to the input of an inverter 323 whose output is connected to a charging circuit 324. The output of charging circuit 324 is connected to a junction 325, the junction 325 being connected to an electronic switch 326, the capacitor 316 and the input of comparator 322.

Charging circuit 324 may be a D—C/D—C converter for charging the capacitor 316 to the desired cardioverting energy level. Alternatively, a series of batteries sufficient to supply the required voltage level may be used directly to eliminate the voltage converter. If the batteries have an internal impedance that would prevent the delivery of the stimulation current directly, a filter or storage capacitor may be placed in parallel with the batteries, in known manner. Our studies indicate that a 50 µF capacitor charged to 1000 volts works well to deliver the necessary energy levels. Also, switch 326 is preferably a triggerable solid state switch controlled to deliver the energy stored in capacitor 316 when triggered to the terminals 22a and b. Preferably, this is accomplished with an SCR arrangement. Also, it is known to the prior art that a truncated capacitor discharge resulting in a trapezoidal signal waveform (as by interrupting the delivery of the energy to the heart before the entire charge has been delivered) is preferable in a cardioverting context. Such an arrangement is disclosed in US—A—3,805,795 which is hereby incorporated by reference.

In operation, the illustrative embodiment of the therapy means 20 of FIG. 7 establishes in response to the application of the preparatory signal on line 18', a defibrillating level charge on capacitor 316 so as to be prepared to deliver a high energy level defibrillating signal across the terminals 22a and b on the appearance of the first high TRIG signal from the patient control means on line 17' (see FIG. 5). In particular, the line 18' is coupled as one input of an AND gate 330, the other input being derived from an inverter 323. Thus, the SENSE signal as derived from the monitoring circuit 14 of FIG. 3 is used to enable the AND gate 330 to permit the charging circuit 324 to charge the capacitor 316. Assuming a charge on capacitor 316 below that desired, the output of the comparator 322 is low causing the junction 321 to be low and one input of AND gate 320 to be low, thereby effectively preventing the output of AND gate 320 from going high. The low signal at junction 321 is inverted by inverter 323 to result in a high input to the AND gate 330. Assuming the presence on a high signal on line 18' to enable the AND circuit 330, a high signal is applied to the charging circuit 324, causing the capacitor 316 to charge under its control in a known manner. When the charge on capacitor 316 reaches the desired level, the output of comparator 322 will go high resulting in a high input to AND gate 320 and a low input to charging circuit 324. The low input to charging circuit 324 causes it to stop charging the capacitor 316, in known manner, while the high input to AND gate 320 allows a high TRIG signal on line 17' to cause the output of AND gate 320 to go high. When the output of AND gate 320 is high, switch 326 is on connecting the capacitor 316 across the output terminals 22a and b. As noted above, switch 326 should be controlled to maintain the on state during the output pulse independently of the output of AND gate 320. An SCR arrangement is suitable for this purpose.

In the overall operation of the therapy means 20 of FIG. 7, a SENSE signal is applied via line 18' to enable the AND gate 330, permitting the charging circuit 324 to charge the capacitor 316. It takes the circuit 324 approximately 15 seconds to charge the capacitor 316 to the 1000 volt level. After the delay imparted by the memory means 28 when the TRIG input signal on line 17' goes high as a result of normal heart activity ceasing and the patient 12 taking appropriate action on the patient control means 30 via one of the circuits of FIGS 5A through 5D or 6A through 6D, the switch 326 is activated to discharge the capacitor 316 through the heart of the patient 12. The capacitor 316 discharges through the patient's heart until the capacitor 316 voltage is reduced to approximately 400 volt level, whereupon the switch 326 becomes inactive again. The resultant cardioverting pulse is a truncated capacitive discharge waveform having a peak magnitude of 1000 volts, and being truncated at the 400 volt level.

If this first cardioverting pulse stimulates normal heart activity, the monitoring circuit 14 of FIG. 3 senses the resumed heart activity, the SENSE signal goes low, and the TRIG signal goes low. The therapy thus ceases. However, if this first pulse does not stimulate normal heart activity, a second cardioverting pulse is needed. Assuming that normal heart activity has not been restored, the control circuit 146 (FIG. 3) becomes active again 5 seconds after the cardioverting pulse, since the contraction sensing portion 36 of the monitoring circuit 56 responds to the heart contraction caused by the cardioverting pulse.

While specific embodiments of the invention have been illustrated and described herein, it is realized that modifications and changes will occur to those skilled in the art.

## Claims

1. A body-implantable therapy device which comprises:

body function monitoring means (14) for generating a signal indicating the apparent existence of a condition requiring therapy;

means (20) for administering therapy appropriate for the condition;

control means (30) operable by the patient for controlling said therapy means to administer or not to administer therapy;

and means (24) for alerting a patient to imminent operation of said therapy means (20) in response to a signal from said monitoring means (14) indicating the apparent existence of a condition requiring therapy,

characterized in that said alerting means (24) are functioning whenever a signal from said monitoring means (14) indicates the existence of a condition requiring therapy and in that memory means (28) are provided giving the patient for a predetermined time the possibility to interrupt the action of said control means (30).

2. A body-implantable therapy device according to claim 1, characterized in that said control means (30) after said predetermined time controls said therapy means (20) not to administer therapy if the patient does not take a defined action in order to enable the control means (30) to permit the therapy means (20) to be actuated (first mode).

3. A body implantable therapy device according to claim 1, wherein said memory means (28) comprises time delay means for triggering said control means (30) and thereby said therapy means (20) said predetermined time after said monitoring means (14) has generated a signal and wherein said control means comprises interrupt means (30) operable by the patient within said predetermined time for inhibiting operation of said therapy means (second mode).

4. A body-implantable therapy device according to claim 3, wherein said interrupt means (30) includes a magnetically operated switch (236) for receiving a command from the patient to inhibit operation of said therapy means.

5. A body-implantable therapy device according to claim 3, wherein said interrupt means (30) includes an electromagnetic field responsive device (238) for receiving a command from the patient to inhibit operation of said therapy means.

6. A body-implantable therapy device according to claim 3, wherein said interrupt means (30) includes a mechanical shock sensor (242) for receiving a command from the patient to inhibit operation of said therapy means.

7. A body-implantable therapy device according to claim 3, wherein said interrupt means (30) is arranged to require periodic positive patient actions for continued inhibiting of the operation of said therapy means (20).

8. A body-implantable therapy device according to one of claims 1 to 7, wherein said monitoring means (28) is designed for generating a signal indicating the apparent existence of a life-threatening arrhythmic heart condition, and said therapy means (20) is a cardioversion means for administering a cardioverting shock to the heart when triggered.

9. A body-implantable therapy device according to one of claims 1 to 8, wherein said alerting means (24) comprise a tissue stimulating device.

## Revendications

1. Dispositif thérapeutique implantable dans le corps qui comporte: un dispositif (14) de contrôle d'une fonction du corps produisant un signal indiquant l'existence apparente d'une condition nécessitant une thérapeutique; un dispositif (20) destiné à administrer une thérapeutique appropriée pour la condition; un dispositif de commande (30) pouvant être manoeuvré par le patient pour commander ledit dispositif thérapeutique afin qu'il administre ou non une thérapeutique; et un dispositif (24) destiné à alerter le malade du fonctionnement imminent dudit dispositif thérapeutique (20) en réponse à un signal provenant dudit dispositif de contrôle (14) indiquant l'existence apparente d'une condition nécessitant une thérapeutique, caractérisé en ce que ledit dispositif d'alerte (24) fonctionne lorsqu'un signal provenant dudit dispositif de contrôle (14) indique l'existence d'une condition nécessitant une thérapeutique et en ce qu'une mémoire (28) est prévue, donnant au malade pendant un temps prédéterminé la possibilité d'interrompre l'action dudit dispositif de commande (30).

2. Dispositif thérapeutique implantable dans le corps selon la revendication 1, caractérisé en ce que ledit dispositif de commande (30), après ledit temps prédéterminé, commande ledit dispositif thérapeutique (20) pour ne pas administrer une thérapeutique si le malade n'entreprend pas une action définie pour autoriser le dispositif de commande (30) à permettre que le dispositif thérapeutique (20) soit actionné dans un premier mode.

3. Dispositif thérapeutique implantable dans le corps selon la revendication 1, dans lequel ladite mémoire (28) consiste en un circuit à retard qui déclenche ledit dispositif de commande (30), et par conséquent ledit dispositif thérapeutique (20), ledit temps prédéterminé après que le dispositif de contrôle (14) a produit un signal, et dans lequel ledit dispositif de commande comporte un dispositif d'interruption (30) pouvant être manoeuvré par le malade dans ledit temps prédéterminé pour empêcher le fonctionnement dudit dispositif thérapeutique dans un second mode.

4. Dispositif thérapeutique implantable dans le corps selon la revendication 3, dans lequel ledit dispositif d'interruption (30) comporte un commutateur (236) actionné magnétiquement pour recevoir une commande du malade et empêcher le fonctionnement dudit dispositif thérapeutique.

5. Dispositif thérapeutique implantable dans le corps selon la revendication 3, dans lequel ledit dispositif d'interruption (30) comporte un dispositif (238) sensible à un champ électromagnétique pour recevoir une commande du malade et empêcher le fonctionnement dudit dispositif thérapeutique.

6. Dispositif thérapeutique implantable dans le corps selon la revendication 3, dans lequel ledit dispositif d'interruption (30) comporte un capteur de choc mécanique (242) pour recevoir une commande du malade et empêcher le fonctionnement dudit dispositif thérapeutique.

7. Dispositif thérapeutique implantable dans le corps selon la revendication 3, dans lequel ledit dispositif d'interruption (30) est agencé pour nécessiter des actions positives periodiques du malade pour continuer à empêcher le fonctionnement dudit dispositif thérapeutique (20).

8. Dispositif thérapeutique implantable dans le corps selon l'une des revendications 1 à 7, dans lequel ledit dispositif de contrôle (28) est conçu pour produire un signal indiquant l'existence apparente d'une condition d'arythmie cardiaque dangereuse pour la vie, et ledit dispositif thérapeutique (20) consiste en un dispositif de rétablissement cardiaque destiné à administrer un choc de rétablissement cardiaque au coeur lorsqu'il est déclenché.

9. Dispositif thérapeutique implantable dans le corps selon l'une des revendications 1 à 8, dans lequel ledit dispositif d'alerte (24) consiste en un dispositif de stimulation de tissus.

**Patentansprüche**

1. Körperimplantierbares Therapiegerät mit einer Körperfunktionsüberwachungseinrichtung (14) zum Erzeugen eines Signals, das das offensichtliche Vorliegen eines Therapie erfordernden Zustands anzeigt;

einer Anordnung (20) zur Durchführung einer für den Zustand geeigneten Therapie;

einer vom Patienten betätigbaren Steuereinrichtung (30) zum Steuern der Therapieanordnung derart, daß diese die Therapie durchführt oder nicht durchführt;

und einer Anordnung (24), die einen Patienten auf ein bevorstehendes Arbeiten der Therapieanordnung (20) aufgrund eines von der Überwachungseinrichtung (14) kommenden Signals aufmerksam macht, welches das offensichtliche Vorliegen eines Therapie erfordernden Zustands anzeigt,

dadurch gekennzeichnet, daß die aufmerksam machende Anordnung (24) immer dann in Funktion tritt, wenn ein Signal von der Überwachungseinrichtung (14) das Vorliegen eines Therapie erfordernden Zustands anzeigt, und daß eine Speicheranordnung (28) vorgesehen ist, die dem Patienten für eine vorbestimmte Zeit die Möglichkeit gibt, die Wirkung der Steuereinrichtung (30) zu unterbrechen.

2. Körperimplantierbares Therapiegerät nach Anspruch 1, dadurch gekennziechnet, daß die Steuereinrichtung (30) nach der vorbestimmten Zeit die Therapieanordnung (20) dahingehend steuert, keine Therapie durchzuführen, wenn der Patient nicht in definierter Weise tätig wird, um es der Steuereinrichtung (30) zu erlauben, ein Tätigwerden der Therapieanordnung (20) zu gestatten (erste Betriebsweise).

3. Körperimplantierbares Therapiegerät nach Anspruch 1, wobei die Speicheranordnung (28) Zeitverzögerungsmittel aufweist, um die Steuereinrichtung (30) und dadurch die Therapieanordnung (20) die vorbestimmte Zeit nach dem Erzeugen eines Signals durch die Überwachungseinrichtung (14) auszulösen, und wobei die Steuereinrichtung Unterbrechungsmittel (30) aufweist, die von dem Patienten innerhalb der vorbestimmten Zeit betätigbar sind, um das Arbeiten der Therapieanordnung zu verhindern (zweite Betriebsweise).

4. Körperimplantierbares Therapiegerät nach Anspruch 3, wobei die Unterbrechungsmittel (30) einen magnetisch betätigten Schalter (236) für den Empfang eines Befehls von dem Patienten, das Arbeiten der Therapieanordnung zu verhindern, aufweisen.

5. Körperimplantierbares Therapiegerät nach Anspruch 3, wobei die Unterbrechungsmittel (30) eine auf ein Elektromagnetfeld ansprechende Einrichtung (238) für den Empfang eines Befehls von dem Patienten, das Arbeiten der Therapieanordnung zu verhindern, aufweisen.

6. Körperimplantierbares Therapiegerät nach Anspruch 3, wobei die Unterbrechungsmittel (30) einen mechanischen Stoßsensor (242) für den Empfang eines Befehls von dem Patienten, das Arbeiten der Therapieanordnung zu verhindern, aufweisen.

7. Körperimplantierbares Therapiegerät nach Anspruch 3, wobei die Unterbrechungsmittel (30) derart ausgelegt sind, daß für ein fortgesetztes Verhindern des Arbeitens der Therapieanordnung (20) periodisches bewußtes Tätigwerden des Patienten erforderlich ist.

8. Körperimplantierbares Therapiegerät nach einem der Ansprüche 1 bis 7, wobei die Überwachungseinrichtung (28) derart ausgelegt ist, daß sie ein Signal erzeugt, welches das offensichtliche Vorliegen einer lebensbedrohenden Herzarrhythmie anzeigt, und die Therapieanordnung (20) eine Kardiovertiereinrichtung ist, die, wenn sie ausgelöst wird, dem Herzen einen Kardiovertierenden Schock zuführt.

9. Körperimplantierbares Therapiegerät nach einem der Ansprüche 1 bis 8, wobei die aufmerksam machende Anordnung (24) ein Gewebestimulationsgerät aufweist.

*FIG. 1.*

MONITORING MEANS `14`

MEMORY MEANS `28`

CONTROL CKT. `30`

THERAPY (STIMULATOR) `20`

PATIENT ALERT `24`

PATIENT `12`

*FIG. 2.*

*FIG. 4.*

MEMORY

30 SECOND RETRIGGERABLE ONE SHOT `172`

SENSE

PATIENT ALERT

POCKET OUTPUT ELECTRODES

+4 V

+6V

TRIG

FIG. 3.

MONITORING CIRCUIT

FIG. 5A.

FIG. 5B.

FIG. 5C.

FIG. 5D.

0 038 080

FIG. 6A.

FIG. 6B.

FIG. 6C.

FIG. 6.D

FIG. 7.

3